# EUROPEAN PATENT SPECIFICATION

(11) **EP 3 782 546 B1**
(45) Date of publication and mention of the grant of the patent: **28.02.2024**
(21) Application number: 19192623.7
(22) Date of filing: 20.08.2019
(51) Int. Cl.: A61H 39/02

(54) **METHOD FOR IDENTIFYING AN ACUPUNCTURE POINT AND/OR A MERIDIAN**
VERFAHREN ZUR IDENTIFIZIERUNG EINES AKUPUNKTURPUNKTES UND/ODER EINES MERIDIANS
PROCÉDÉ D'IDENTIFICATION D'UN POINT D'ACUPUNCTURE ET/OU DE MÉRIDIEN

(43) Date of publication of application: 24.02.2021
(73) Proprietor: Rayonex Biomedical GmbH, 57368 Lennestadt (DE)
(72) Inventor: HEIMES, Dietmar, 57368 Lennestadt (DE)
(74) Representative: König Szynka Tilmann von Renesse Patentanwälte Partnerschaft mbB Düsseldorf

(56) References cited:
- WO-A1-2016/128985
- WO-A2-2005/086725
- CN-A- 108 403 413
- DE-A1-102017 005 051
- US-A- 5 339 827
- Rayonex: "4. User Manual of the Rayocomp PS 1000 Polar Contents", , 10 August 2010 (2010-08-10), pages 1-99, XP055132394, Internet Retrieved from the Internet: URL:http://www.extrao.fr/index.php/image/n ews/76/9000EN_FB069_4_Benutzerhandbuch_PS1 000polar_Rev3.pdf [retrieved on 2014-07-30]

## Description

### FIELD OF THE INVENTION

The invention relates to the field of modernized Traditional Chinese Medicine and to a method for identifying an acupuncture point and/or a meridian of a subject by using electrophysiological data obtained from the subject. The invention also relates to a medical device for identifying an acupuncture point and/or a meridian and to the use of a medical device for identifying an acupuncture point and/or a meridian.

### BACKGROUND OF THE INVENTION

The Traditional Chinese Medicine (TCM) describes a network known as "meridians" through which a subject's life force or life energy ("qi") flows. If there is an imbalance of the *qi,* the subject may become ill. The *qi* may be tonified or sedated by acupuncture to balance the *qi* depending on the subject's needs. There are twelve standard meridians (principal meridians) and eight extraordinary meridians. Each of the twelve principal meridians is associated with an organ, such as the heart, pericardium, lung, spleen, liver, kidney (the yin organs) or stomach, gall-bladder, large and small intestine, urinary bladder and tri-heater or triple burner (the yang organs). The tri-heater or triple burner is viewed in the concept of TCM as one of the twelve vital organs, it regulates the flow of *qi* through the organs. Acupuncture points are located along the meridians. More than four hundred acupuncture points are known. Most of them are located along the principal meridians.

In traditional acupuncture, the acupuncturist selects which points to treat, in particular which points to stimulate (tonify) or sedate, by observing and questioning the patient. The observing and questioning can be divided into inspection, auscultation and olfaction, inquiring, and palpation. Inspection focuses on the face and particularly on the tongue, including analysis of the tongue size, shape, tension, color and coating, and the absence or presence of teeth marks around the edge. Auscultation and olfaction involve listening for particular sounds such as wheezing, and observing body odor. Inquiring involves focusing on the "seven inquiries": chills and fever; perspiration; appetite, thirst and taste; defecation and urination; pain; sleep; and menses and leukorrhea. Palpation is focusing on feeling the body for tender *"A-shi"* points (points of sensitivity or discomfort) and feeling the pulse. This concept is called "pattern differentiation", or "zheng" (Yang et al.: "The Exploration of Disease Pattern, Zheng, for Differentiation of Allergic Rhinitis in Traditional Chinese Medicine Practice," Evidence-Based Complementary and Alternative Medicine, Vol. 2012, p. 1-7, 2012).

However, this method of selecting, i. e. identifying individual acupuncture points and/or meridians is very laborious, complex and subjective because it depends on the TCM practitioner and his experience. Finding ways to incorporate TCM knowledge into clinical practice and eliminating variability is thus an important issue in modernizing TCM.

There are attempts to standardize pattern differentiation. These approaches are, however, still laborious and complex (Cheng et al. "Biologic basis of TCM syndromes and the standardization of syndrome classification, Journal of Traditional Chinese Medical Sciences, Vol. 1(2), p. 92-97, 2014). Such approaches also necessitate a TCM practitioner.

Thus, there is a need for a simple and objective method for identifying an acupuncture point and/or a meridian of a subject. US5339827A discloses a device for helping find acupuncture points on a human patient and the recording, storage and analysis of the electrical impedance of the points after they are found, including (i) a probe electrode having a conductive end; (ii) a reference electrode; (iii) oscillator means to generate an oscillatory waveform with a high content of harmonic components; (iv) an impedance-to-frequency converter means; (v) a computer system means electrically connected to the impedance-to-frequency converter means to display the impedances as they are being measured by the probe electrode on a screen; the computer system means having a display screen, digital memory and programmed software means to analyze the impedances at the found acupuncture points.

### SUMMARY OF THE INVENTION

The invention is set out in the appended set of claims.

### SUMMARY OF THE DISCLOSURE

It is thus an object of the present invention to provide an objective method for identifying an acupuncture point and/or a meridian of a subject.

It was surprisingly found that by
a) detecting electrophysiological data points of the subject as a function of electromagnetic frequencies imposed on the subject,
b) comparing these electrophysiological data points with a standard value for each of these electrophysiological data points,
c) identifying at least one of the frequencies from step a) at which the electrophysiological data point deviates from the standard value (resonance frequency),
   it is possible to
d) assign an acupuncture point and/or a meridian to the resonance frequency, and
e) therewith individualize for said subject a set of at least one acupuncture point and/or meridian.

This method provides a simple, reliable and objective way to for identifying an acupuncture point and/or a meridian of a subject. There is no need for a TCM practitioner as any person trained in acquiring electrophysiological data of a patient can perform the method. The method can for example be performed with a bioresonance device. Such a bioresonance device is for example described in EP 2 799 110 A1. Hence, for example any person familiar with a biresonance device can perform the method. The output is a set of at least one acupuncture point and/or meridian which is individual for each subject.

Based on the identified set of at least one acupuncture point and/or meridian the TCM practitioner can make a decision on which one of the identified acupuncture points and/or meridians to select for a potential further treatment. Thus, the identified acupuncture points and/or meridians do not allow a diagnosis *per se* but provide an objectively obtained preselection for the TCM practitioner. This preselection also does not constitute a therapy, i. e. the inventive method does not comprise a treatment step; rather, the method allows for a possible future treatment decision.

### BRIEF DESCRIPTION OF THE FIGURES

- Fig. 1: Flowchart of the method according to the present invention
- Fig. 2: Assignment of resonance frequencies to meridians and acupuncture points on the lung meridian
- Fig. 3: Assignment of resonance frequencies to meridians and acupuncture points on the liver meridian
- Fig. 4: Display of a bioresonance device showing the resonance frequencies that have been determined
- Fig. 5: Display of a bioresonance device showing the most prominent meridians that have been determined
- Fig. 6: Display of a bioresonance device showing acupuncture points on the lung meridian which need to be sedated or tonified

### DETAILED DESCRIPTION OF THE INVENTION

In a first aspect of the invention, the invention relates to a method for identifying an acupuncture point and/or a meridian of a subject comprising the following steps:
a) detecting electrophysiological data points of the subject as a function of electromagnetic frequencies imposed on the subject,
b) comparing these electrophysiological data points with a standard value for each of these electrophysiological data points,
c) identifying at least one of the frequencies from step a) at which the electrophysiological data point deviates from the standard value (resonance frequency),
d) assigning an acupuncture point and/or a meridian to the resonance frequency, and
e) therewith individualizing for said subject a set of at least one acupuncture point and/or meridian.

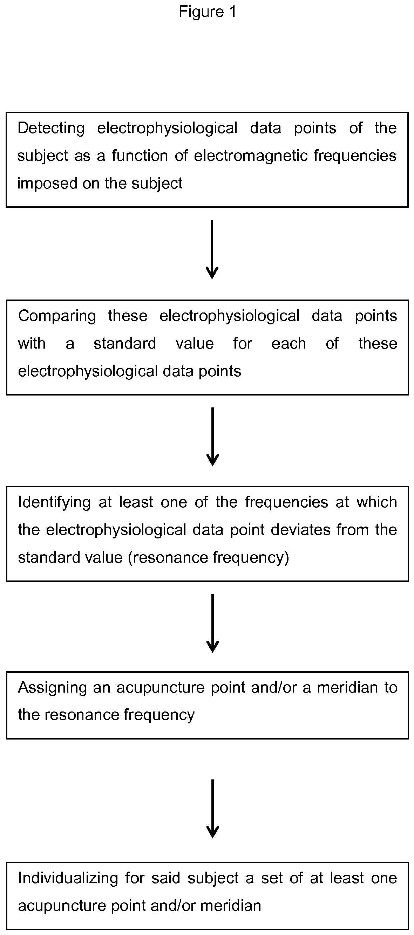

The method is also illustrated in the flowchart of Figure 1. In one embodiment, the steps a) to c) are repeated.

In a second aspect, the invention relates to a medical device, preferably a bioresonance device, for identifying an acupuncture point and/or a meridian of a subject comprising the following modules:
- a module for recording electrophysiological data points of a subject
- a memory module comprising a database of resonance frequencies and of the corresponding acupuncture points,
- an electromagnetic frequency generator module; and
- at least one electrode used both for recording the electrophysiological data points and for imposing the electromagnetic frequencies on the subject.

In a third aspect, the invention relates to a use of a medical device which records electrophysiological activity, preferably a bioresonance device, for identifying an acupuncture point and/or a meridian of a subject.

The inventors have found that electrophysiological activity can be used to identify an acupuncture point and/or a meridian and thereby to determine an individual set of acupuncture points and/or meridians of a subject. This method thus allows a simple, accurate and objective preselection of acupuncture points and/or meridians. The individualized set of acupuncture points and/or meridians may differ depending on the subject and its condition. The TCM practitioner can then make a decision which one/s of the identified acupuncture points and/or meridians to select for a potential further treatment.

It is not necessary to perform laborious tests in order to acquire the information on the acupuncture point/s and/or meridian/s. It is also not necessary to observe and question the patient. After the identification of possible acupuncture points and/or meridians it is advisable to consult a TCM practitioner who can then decide how to use and process the retrieved data.

It is also possible to identify only one acupuncture point, which is the most important acupuncture point. This is extremely helpful for the TCM practitioner because the most important acupuncture point is laborious to identify with traditional methods. The TCM practitioner can then decide whether and how this identified acupuncture point shall be treated.

In one embodiment, the subject is a human being or an animal. An animal is for example a vertebrate, in particular a mammal, in particular a horse, a cat or a dog. In a preferred embodiment, the animal is a horse. In another preferred embodiment, the animal is a dog. In another preferred embodiment, the subject is a human being.

The subject may also be termed a "patient". However, the subject can be in any condition. In particular, the subject may be healthy or affected with disease or ill health. Hence, the term "patient" does not necessarily mean that the subject is ill.

In the sense of the present invention, an "acupuncture point" means a site on the body which is used in acupuncture, acupressure, or other treatment systems based on TCM. A therapeutic effect usually requires the selection/identification of an acupuncture point on a subject and the stimulation or sedation of this acupuncture point.

A "meridian" in the sense of the present invention means a pathway along a subject's body which is used in acupuncture, acupressure, or other treatment systems based on TCM. Typically, acupuncture points are located along the meridians.

The present invention is based on the knowledge that a human or animal organism physiologically reacts to a stimulus in the form of an electromagnetic frequency acting on the organism or applied to the organism ("imposed") and that the sum of possible different reactions is detectable as a change of the detected electrophysiological data points. Depending on the initial physiological state of the organism, the extent of change in the electrophysiological data points as a function of the imposed frequency may vary.

The inventors have thus established that the deviation of an electrophysiological data point from a standard value can be used to determine resonance frequencies of a subject which are suitable for providing information on the physiological condition of this subject. In one embodiment, the determined resonance frequencies form a resonance frequency pattern.

Preferably, at least two or more electrophysiological data points are detected.

In one embodiment, the electrophysiological data points are detected on the basis of a method selected from the group comprising electroencephalography, electrocardiography, electrogastrography, electrocochleography, electronystagmography, electrooculography, electroretinography, electromyography, and electroneurography. The method of electrocardiography (ECG) is preferred.

All of these electrophysiological methods have in common that the electrical activity, i.e. the conduction of an electrical impulse or excitation, of an organ or tissue is recorded. This activity is measured over time and recorded in curves typical for the respective method. The measured electrophysiological data points are thus typically recorded as curves. The shape of the respective curve, e.g. the position and slope of certain peaks, provides information about the physiological condition of the respective organ or tissue of the subject.

In an "electrocardiogram", for example, a heartbeat is represented in a typical curve known as the P-Q-R-S-T-U curve. The individual letters are designations for characteristic peaks, valleys or other typical features of the curve. The person skilled in the art is familiar with such ECG curves and their analysis.

The detected electrophysiological data points are preferably not used directly (in terms of the raw data) but computationally processed first, i. e. data points calculated from the measured electrophysiological data points can be used for the comparison with a standard value. Thus, in one embodiment, the detected electrophysiological data points are used for further calculations and the calculated data points are compared with a standard value for these electrophysiological data points.

It is particularly preferred to calculate the area under a curve (AUC). i. e. preferably the further calculation is the calculation of the AUC, wherein the curve is preferably assembled from the detected electrophysiological data points. The person skilled in the art knows how to read ECG curves and how to calculate the AUC. Usually, a curve section is first selected before the AUC is calculated. For example, the P-Q-R-S-T-U curve measured in an electrocardiogram preferably defines a curve section (physiologically the electrical conduction of a heartbeat). In this case, the AUC of a P-Q-R-S-T-U curve measured at a certain frequency is determined as the electrophysiological data point.

Other characteristics of the detected electrophysiological data points or curves may also be used. For example, if an ECG is recorded, the slope of the Q-R-S peak ("gradient") may be calculated. This gradient can be described by an angle alpha which is the angle between the perpendicular to the X-axis (= time axis in the ECG) and the gradient arm of the R-peak. In a particularly preferred embodiment, both the angle alpha as well as the AUC are calculated and used as the computationally processed electrophysiological data points.

In a further embodiment, alternatively or in addition to the AUC or the gradient one or more of the following data points may be used as an electrophysiological data point: heart rate, heart rhythm, length, amplitude or shape of the P-wave, the T-wave, the U-wave or the QRS complex, length of the PQ interval, the QT interval, the RR interval or the ST distance. In a preferred embodiment, the heart rate, in particular the heart rate variability (HRV) is determined. The HRV can for example be determined on the basis of three to four heartbeats. Three to four heartbeats are advantageous, as the HRV only reacts briefly to the external (imposed) frequencies and adapts to the new situation after three to four heartbeats at the latest.

Thus, in one embodiment, an electrocardiogram is recorded and the electrophysiological data points are selected from the group comprising area under the P-Q-R-S-T-U curve, slope of the Q-R-S peak and heart rate variability, preferably the area under the P-Q-R-S-T-U curve.

The values calculated from the measured electrophysiological data points may also be referred to as "computationally processed data points". However, the term "electrophysiological data points" covers both the measured electrophysiological data points and the computationally processed data points calculated from the measured electrophysiological data points.

Typically, at least one electrode is placed over the skin of the subject for recording electrical activity. In one embodiment, a measuring electrode is used for recording the electrophysiological data points. In a preferred embodiment, the measuring electrode is comprised in a clip that can be attached to the finger, toe or ear (especially for pulse oximetry) of a subject, in a (compressible) cuff, a pad, a chest belt or a pulse watch that can be attached to or worn by a subject. Preferably, at least one pad is used comprising an electrode. The pad is typically connected to an electrode cable.

The electrophysiological data points are preferably detected (recorded) as a function of the electromagnetic oscillations (frequencies) imposed on the subject. This means that the data points are preferably recorded while the subject receives the electrical impulses, i.e. the stimulation, from the imposed frequencies. In this embodiment, the subject is preferably simultaneously connected to a device for recording the electrophysiological data points and to a device for imposing (stimulating) the frequencies on the subject. Both devices can be separate or combined. Preferably, one electrode is used both for recording the electrophysiological data points and for imposing the electromagnetic frequencies on the subject. Such an electrode which is used both for recording and for imposing electromagnetic frequencies can be comprised in a clip that can be attached to the finger, toe or ear (especially for pulse oximetry) of a subject, in a (compressible) cuff, a pad, a chest belt or a pulse watch that can be attached to or worn by a subject. Preferably, at least one pad is used comprising an electrode. The pad is typically connected to an electrode cable.

According to the invention, at least two frequencies, in particular more than two frequencies, are imposed on the subject. In one embodiment, the more than two frequencies are imposed as a sequence of individual frequencies, i.e. the frequencies act on the subject one after the other and not as a mixture of different frequencies. Thereby, the effect of a single frequency on the subject can be determined.

In one embodiment, between 10 and 500 frequencies, preferably between 50 and 400, and more preferably between 100 and 200 frequencies are imposed on the subject. Preferably, at each of these frequencies, an electrophysiological data point, preferably a curve, is recorded. Hence, at N imposed frequencies, N electrophysiological data point curves may be recorded.

The frequencies applied preferably lie in the range of 0.1 Hz to 10 GHz. In a preferred embodiment, they lie in the range of 0.1 Hz to 500 MHz and preferably in the range of 0.1 Hz to 100 MHz. If the frequencies are imposed sequentially, i.e. in a sequence, they can have the same frequency difference to each other, i.e. the frequency sequence preferably has a uniform step size. For example, if a sequence of frequencies with a frequency value of 0.5 kHz; 1.0 kHz; 1.5 kHz; 2.0 kHz; 2.5 kHz; 3.0 kHz; 3.5 kHz; 4.0 kHz etc. is used, the step size is 0.5 kHz.

In a preferred embodiment, the range of frequencies can be adjusted according to the user's needs.

However, in one embodiment it is also possible to apply specific frequencies (instead of a range of frequencies), in particular to apply the individual frequency or frequencies that can be assigned to an acupuncture point and/or to a meridian.

Typically, the frequency is generated by a frequency generator module. The terms "electromagnetic frequency generator module" and "frequency generator module" are used synonymously. Preferably, the frequency generator module can generate frequency spectra with different single frequencies. Preferably, the frequency generator module can be regulated.

In a preferred embodiment, the frequency generator module uses the dipole technology. This technology advantageously allows frequencies higher than 100 kHz to be generated.

In one embodiment, individual frequencies are imposed on the subject. However, it is preferable to apply frequency spectra, i.e. to apply several individual frequencies to the subject simultaneously. In a preferred embodiment, the electrophysiological data points according to step a) are detected as a function of sequentially applied frequency spectra.

Preferably, the imposed spectra are decadic frequency spectra, i.e. spectra which frequencies increase by a factor of 10 compared to the respective lower frequency. The lowest frequency may also be termed the "basic frequency value". For example, with a basic frequency value of 0.5 kHz, the decadic frequency spectrum would contain frequencies of 5 kHz, 50 kHz, 500 kHz, etc.

In this preferred embodiment, the basic frequency value is not a specific single frequency, but the smallest value of a frequency spectrum, whereby the other frequencies each have a frequency that is 10ⁿ higher. In the context of the present invention, the general term frequency means both the single frequency and a frequency spectrum.

In a preferred embodiment, the lowest frequency is imposed first. The frequency is then increased step by step up to the highest frequency. However, the sequence can also be exactly the other way round, i.e. it is also possible to start with the highest frequency and then to reduce it step by step to the lowest frequency.

According to step b) of the inventive method, the electrophysiological data points are compared with a standard value for these data points in order to determine at least one resonance frequency (step c)). The resonance frequency is the frequency whose electrophysiological data point deviates from the standard value. When two or more frequencies are imposed, two or more resonance frequencies can be found. Usually, the number of determined resonance frequencies is between 10 and 100, preferably between 50 and 80 resonance frequencies. The number of resonance frequencies determined during a measurement can for example be influenced by the use of a filter.

The standard value can be a value retrieved from an external database, i.e. a value that is not specific for a certain subject. The "typical" (i. e. standard) curves of the electrophysiological data points according to the invention are generally known to the expert. The curve of a subject at a certain frequency can be compared with such a typical curve. The analysis of the AUC can preferably serve as a benchmark for this purpose.

In a preferred embodiment, the standard value is determined by calculating the mean value of all electrophysiological data points of the subject of step a) of the inventive method. Using the mean value from the subject itself has the advantage that it reflects the individual electrophysiologically detectable condition of the subject at the time of measurement. The area below the measured curve is preferably used for this calculation.

The frequencies at which the AUC deviates from the mean value of all recorded AUC would then represent the resonance frequencies. The extent of the deviation that is considered diagnostically or therapeutically relevant can be determined by a healthcare professional. By determining the resonance frequencies, an individual "energetic fingerprint" of the subject is created.

In step d) of the inventive method, an acupuncture point and/or meridian is assigned to the identified resonance frequency/frequencies of step c). This is possible because the corresponding acupuncture point and meridian of each resonance frequency is known.

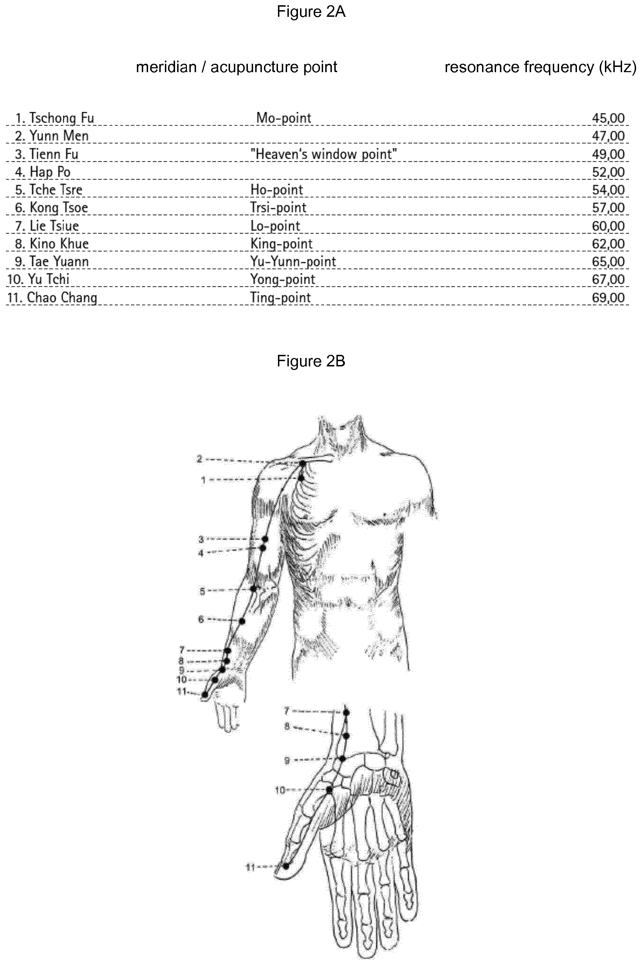

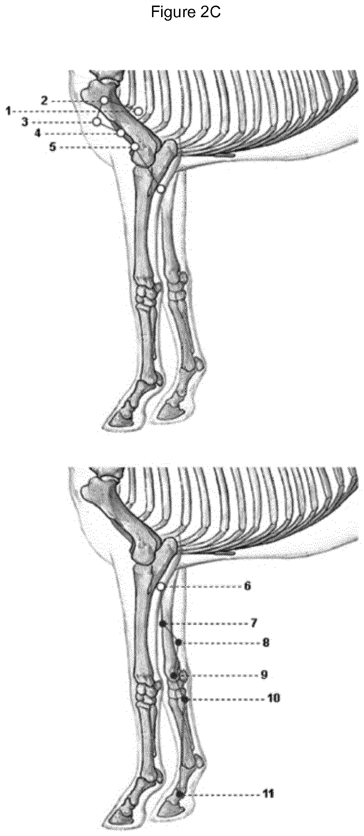

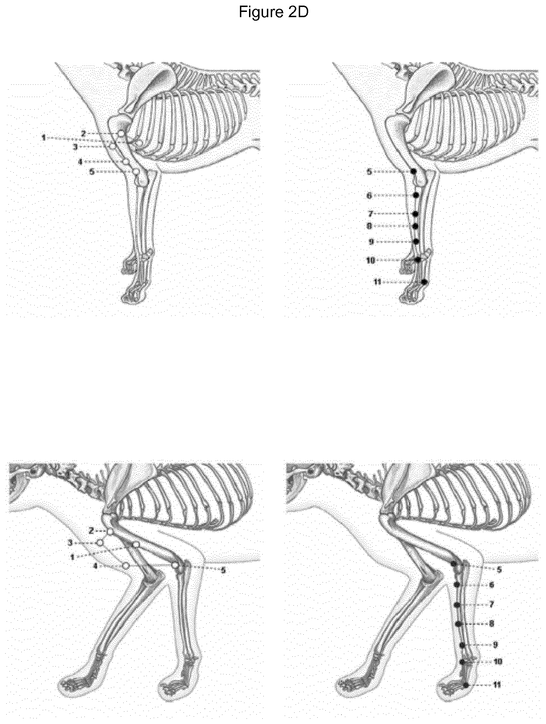

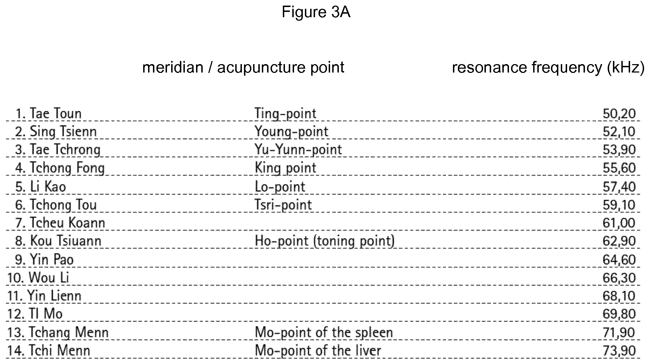

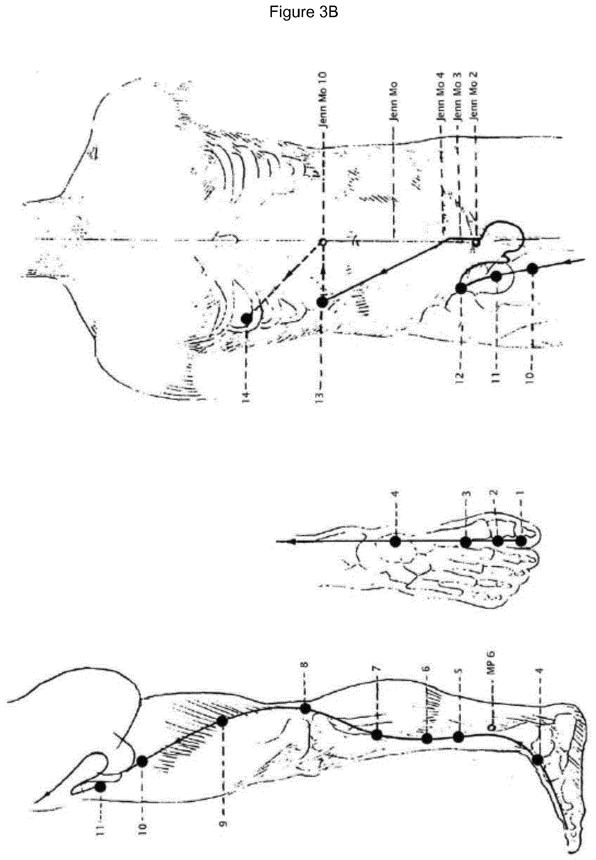

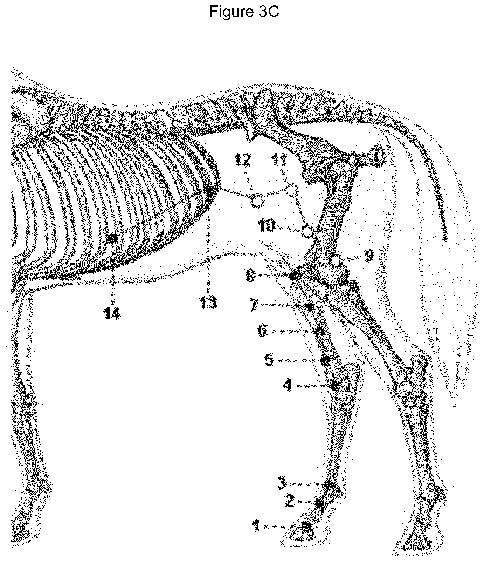

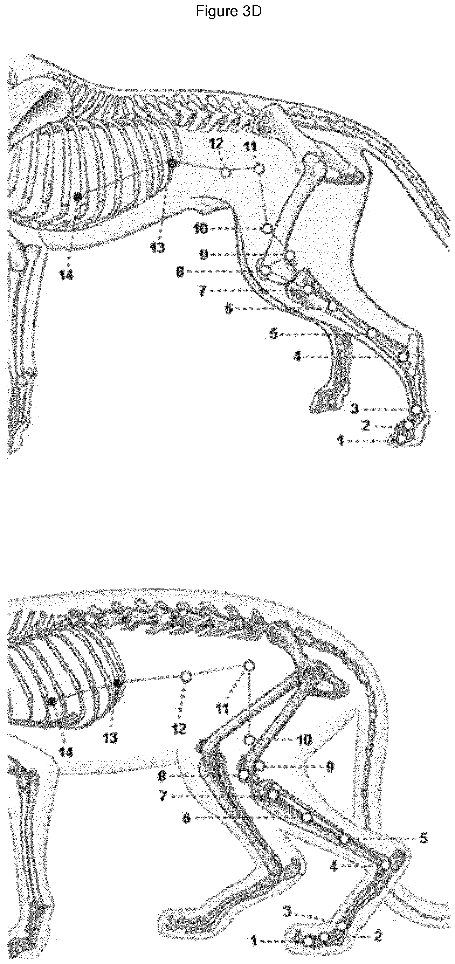

Figures 2 and 3 exemplarily show the assignment of resonance frequencies to meridians and acupuncture points on the meridians (lung and liver, respectively). Figure 2A shows the resonance frequencies which are assigned to acupuncture points on the lung meridian. For example, a resonance frequency of 45.00 is assigned to acupuncture point 1 on the lung meridian ("Tschong Fu" or "Mo-point"). Figure 2B shows the localization of the lung meridian and the respective acupuncture points on the human body. For example, the above-mentioned acupuncture point 1 on the lung meridian is located on the upper lateral chest. Likewise, figures 2C and 2D show the localization of the lung meridian and the respective acupuncture points on the body of a horse (2C), a dog (2D top), or a cat (2D bottom). Figure 3A discloses the assignment of resonance frequencies to acupuncture points on the liver meridian. Figures 3B, 3C and 3D show the localization of the liver meridian and the respective acupuncture points on the body of a human (3B), a horse (3C), a dog (3D top) or a cat (3D bottom).

A list of resonance frequencies and the respective meridians and acupuncture points can for example be found in "Bioresonanz nach Paul Schmidt" (Heimes, D.; 4th edition; 2013; Spurbuchverlag; Annex 24.12).

In one embodiment, the meridian is identified by identifying the acupuncture point and assigning the meridian to the acupuncture point. This is possible because each acupuncture point is typically located on a meridian.

In one embodiment, it is intended to determine whether the acupuncture point and/or meridian assigned in step d) is an acupuncture point and/or meridian to be tonified or an acupuncture point and/or meridian to be sedated. The inventors have found that if the electrophysiological data point at a certain resonance frequency is below a standard value, then the assigned acupuncture point and/or meridian typically needs to be tonified. Likewise, if the electrophysiological data point at a certain resonance frequency is above a standard value, then the assigned acupuncture point and/or meridian typically needs to be sedated. Thus, by determining whether an electrophysiological data point is above or below a standard value, it can be determined whether the acupuncture point and/or meridian assigned is an acupuncture point and/or meridian to be sedated or an acupuncture point and/or meridian to be tonified.

In a particularly preferred embodiment, the inventive method comprises the steps of
a) detecting electrophysiological data points of the subject as a function of electromagnetic frequencies imposed on the subject,
b) comparing these electrophysiological data points with a standard value for each of these electrophysiological data points,
c) identifying at least one of the frequencies from step a) at which the electrophysiological data point deviates from the standard value (resonance frequency),
d) assigning an acupuncture point and/or a meridian to the resonance frequency,
e) therewith individualizing for said subject a set of at least one acupuncture point and/or meridian, and
f) determining whether the acupuncture point and/or meridian assigned in step d) is an acupuncture point and/or meridian to be tonified or an acupuncture point and/or meridian to be sedated
wherein the electrophysiological data points are detected on the basis of electrocardiography.

The invention also relates to a medical device, preferably a bioresonance device, for identifying an acupuncture point and/or a meridian of a subject comprising the following modules:
- a module for recording electrophysiological data points of a subject
- a memory module comprising a database of resonance frequencies and of the corresponding acupuncture points,
- an electromagnetic frequency generator module; and
- at least one electrode used both for recording the electrophysiological data points and for imposing the electromagnetic frequencies on the subject.

A "bioresonance device" is advantageously used for the inventive method. A bioresonance device typically comprises the above modules and allows detecting electrophysiological data points of the subject as a function of electromagnetic frequencies imposed on the subject, comparing these electrophysiological data points with a standard value for each of these electrophysiological data points, identifying at least one of the frequencies at which the electrophysiological data point deviates from the standard value (resonance frequency), assigning an acupuncture point and/or a meridian to the resonance frequency, and therewith individualizing for said subject a set of at least one acupuncture point and/or meridian.

The module for recording electrophysiological data points can be any module which can retrieve and collect the information of electrophysiological data points.

The memory module advantageously comprises a database of resonance frequencies and of the corresponding acupuncture points and allows assigning an acupuncture point and/or a meridian to the respective resonance frequency. Hence, the memory module preferably comprises the data as for example depicted in "Bioresonanz nach Paul Schmidt" (Heimes, D.; 4th edition; 2013; Spurbuchverlag; Annex 24.12) which shows lists of resonance frequencies and the respective meridians and acupuncture points.

The electromagnetic frequency generator module typically allows generating the frequency that is imposed on the subject.

Preferably, the medical device comprises at least one electrode which is used both for recording the electrophysiological data points and for imposing the electromagnetic frequencies on the subject.

In a preferred embodiment, the medical device further comprises a data processing unit. The data processing unit preferably serves for the computational processing which preferably allows identifying resonance frequencies by comparing the electrophysiological data points with a standard value for each of these electrophysiological data points and providing an individualized set of at least one acupuncture point and/or meridian.

In a preferred embodiment, the data processing unit also allows determining whether an electrophysiological data point is above or below a standard value, so that it can be determined whether the acupuncture point and/or meridian assigned is an acupuncture point and/or meridian to be sedated or an acupuncture point and/or meridian to be tonified.

In another preferred embodiment, the medical device further comprises an output unit, preferably a display. The output unit preferably serves for providing the output in user readable form. The user readable form can for example be text, graphics, tactile, audio, and video. The output unit may for example be a printer, a memory device saving the output information, a speaker or a display. Preferably, the output device is a display, such as a touch screen.

In a further embodiment, the medical device comprises an input unit such as a keyboard, preferably a membrane keyboard.

In a particularly preferred embodiment, the medical device comprises
- a module for recording electrophysiological data points of a subject
- a memory module comprising a database of resonance frequencies and of the corresponding acupuncture points,
- an electromagnetic frequency generator module; and
- at least one electrode used both for recording the electrophysiological data points and for imposing the electromagnetic frequencies on the subject
- a data processing unit,
- an input unit, preferably a keyboard, and
- an output unit, preferably a display.

The invention also relates to the use of a medical device which records electrophysiological activity, preferably a bioresonance device, for identifying an acupuncture point and/or a meridian of a subject. The medical device is preferably used for the method according to the present invention.

### EXAMPLES

### Example 1: Using a bioresonance device for the method for identifying an acupuncture point and/or a meridian of a subject

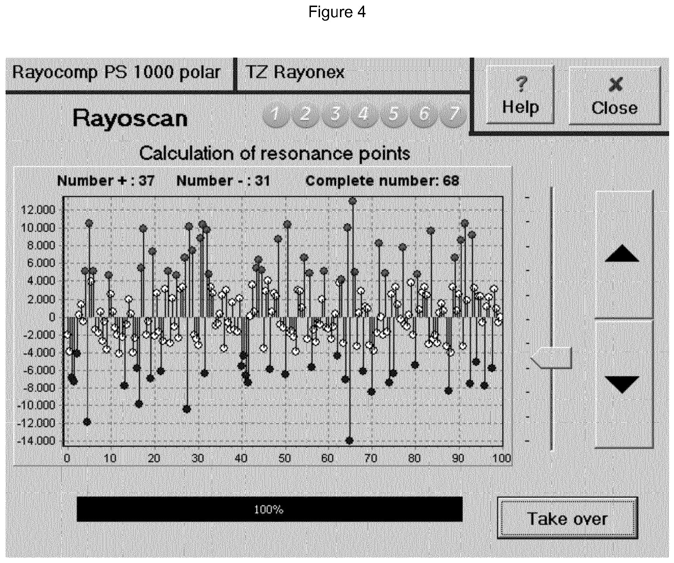

A bioresonance device can be used for the method for identifying an acupuncture point and/or a meridian of a subject. As an output, the bioresonance device displays the resonance frequencies that have been determined (Figure 4). The meridians/acupuncture points assigned to the data points which show values (on the y-axis) above zero, need to be tonified. The meridians/acupuncture points assigned to the data points which show values (on the y-axis) below zero, need to be sedated. A person skilled in the art can set a threshold on the y-axis above which (positive values) or below which (negative values) the resonance frequencies shall be selected (depicted as "full" dots as opposed to "empty" dots).

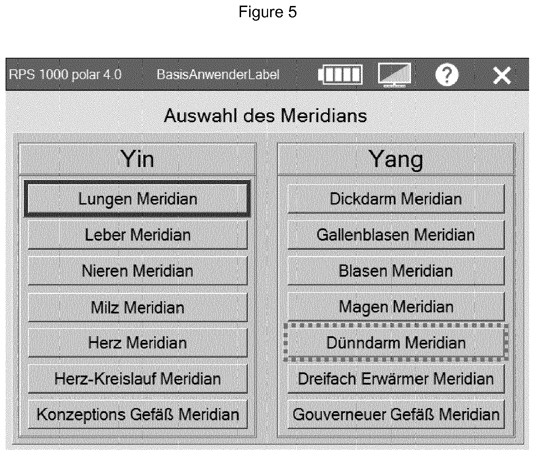

The bioresonance device thus can assign an acupuncture point and/or a meridian to the identified resonance frequency. As an output the device can display the most prominent meridians (Figure 5). On the left, the lung meridian ("Lungen Meridian") is encircled with a solid line. This is an indication that acupuncture point/s on the lung meridian need to be treated. On the right, the small intestine meridian ("Dünndarm Meridian") is encircled with a dotted line. This is an indication that acupuncture point/s on the small intestine meridian need to be treated. The device thus shows the most prominent meridians that may indicate a need to be treated. The final decision whether an acupuncture point and/or meridian needs to be treated is up to the TCM practitioner.

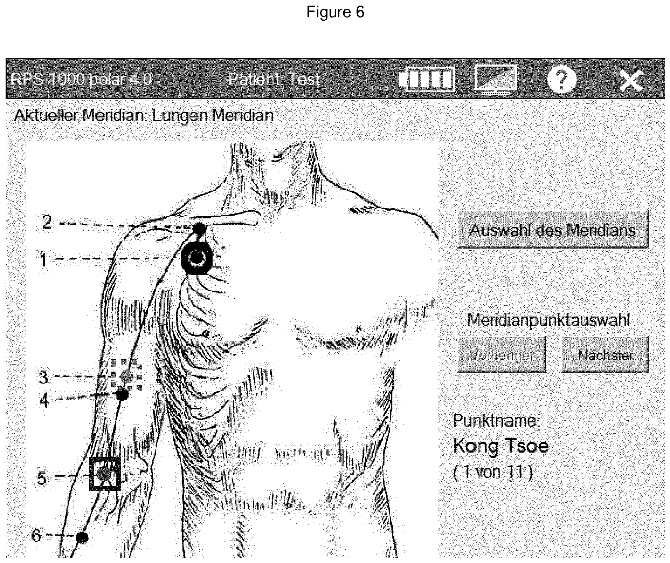

Figure 6 shows distinct acupuncture points on the lung meridian which need to be sedated (Point 5, enframed with a solid square) or tonified (Point 3, enframed with a dotted square).

### Example 2: Comparison of acupuncture points and meridians identified by a method of the state of the art with the method according to the invention (subject: horse)

In this example, a horse was chosen as a subject and acupuncture points and meridians were identified by a method of the state of the art and compared with acupuncture points and meridians identified with the method according to the invention.

The subject is a 10-year-old gelding with hypertonus and multiple tendon damage on the forehand. The owner describes his horse as very powerful, but also dominant and somewhat difficult to handle. The horse is presented because old tendon injuries have caused more difficulties in the last weeks. The horse has also had a conjunctivitis for two days.

### Traditional identification of acupuncture points and meridians

During the diagnosis it is noticeable that the horse smells slightly sour and the eyes water strongly. The entire musculature is well developed, but very tense.

Traditionally, "Shu points" located along the bladder meridian on the back of the horse can be tested for tenderness on palpation. In the current example, the Shu points "bladder 13" "bladder 18" are tender on palpation. The Shu points give information about the disturbed meridians in the organism.

Based on this information, the TCM practitioner has reasoned that for example the liver meridian must now be sedated in order to make the qi flow again and to release the stagnation. The liver manifests itself in the eyes and tendons.

The TCM practitioner has selected the following acupuncture points:
Spleen 21
Lung 7
Lung 11
Colon 4
Stomach 2
Liver 3
Liver 8
Bladder 13
Bladder 18

### Identification of acupuncture points and meridians according to the present invention

The horse is connected with the electrodes of a bioresonance device. At the end of the measurement, the evaluation shows which meridians are energetically disturbed (based on the resonance frequencies identified):

| **Resonance frequency** | **acupuncture points/meridians** |
|---|---|
| 53.90 | liver 3 (s) |
| 62.90 | liver 8 (t) |
| 60.00 | lung 7 (t) |
| 69.00 | lung 11 (t) |
| 19.30 | bladder 13 (t) |
| 20.30 | bladder 18 (s) |
| 5.70 | colon 4 (t) |
| 10.30 | stomach 2 (s) |
| 40.60 | spleen 21 (t) |

In addition, the bioresonance device can reveal which acupuncture points to sedate (depicted as "s") and which ones to tonify (depicted as "t").

As can be seen, the same acupuncture points/meridians were identified independent from the method selected to identify them. The method of the present invention has thus been proven effective in identifying an acupuncture point and/or a meridian.

## Claims

1. Method for identifying an acupuncture point and/or a meridian of a subject comprising the following steps:
a) detecting electrophysiological data points, detected on the basis of an electrophysiological method recording electrical activity of an organ or tissue, of the subject as a function of electromagnetic frequencies imposed on the subject,
b) comparing these electrophysiological data points with a standard value for each of these electrophysiological data points,
c) identifying at least one of the frequencies from step a) as a resonance frequency at which the electrophysiological data point deviates from the standard value,
d) assigning an acupuncture point and/or a meridian to the resonance frequency, and
e) therewith individualizing for said subject a set of at least one acupuncture point and/or meridian,
wherein the method is performed with a medical device, preferably a bioresonance device, for identifying an acupuncture point and/or a meridian of a subject comprising the following modules:
- a module for recording electrophysiological data points of a subject
- a memory module comprising a database of resonance frequencies and of the corresponding acupuncture points,
- an electromagnetic frequency generator module; and
- at least one electrode used both for recording the electrophysiological data points and for imposing the electromagnetic frequencies on the subject.

2. The method according to claim 1, wherein the electrophysiological data points are detected on the basis of a method selected from the group comprising electroencephalography, electrocardiography, electrogastrography, electrocochleography, electronystagmography, electrooculography, electroretinography, electromyography, and electroneurography, preferably electrocardiography.

3. The method according to claim 1 or 2, wherein the detected electrophysiological data points are used for further calculations and the calculated data points are compared with a standard value for these electrophysiological data points.

4. The method according to claim 3, wherein the further calculation is the calculation of the area under a curve and wherein the curve is assembled from the detected electrophysiological data points.

5. The method according to any of the preceding claims, wherein an electrocardiogram is recorded and the electrophysiological data points are selected from the group comprising area under the P-Q-R-S-T-U curve, slope of the Q-R-S peak and heart rate variability, preferably the area under the P-Q-R-S-T-U curve.

6. The method according to any of the preceding claims, wherein the standard value is determined by calculating the mean value of all electrophysiological data points of the subject of step a).

7. The method according to any of the preceding claims, wherein the electrophysiological data points according to step a) are detected as a function of sequentially applied frequency spectra.

8. The method according to any of the preceding claims, wherein the frequencies imposed on the subject are in the range of 0.1 Hz to 100 MHz.

9. The method according to any of the preceding claims, wherein the determined resonance frequencies form a resonance frequency pattern.

10. The method according to any of the preceding claims, wherein the meridian is identified by identifying the acupuncture point and assigning the meridian to the acupuncture point.

11. The method according to any of the preceding claims, further comprising step
f) determining whether the acupuncture point and/or meridian assigned in step d) is an acupuncture point and/or meridian to be tonified or an acupuncture point and/or meridian to be sedated.

12. A medical device, preferably a bioresonance device, for identifying an acupuncture point and/or a meridian of a subject comprising the following modules:
- a module for recording electrophysiological data points of a subject,
- a memory module comprising a database of resonance frequencies and of the corresponding acupuncture points,
- an electromagnetic frequency generator module; and
- at least one electrode used both for recording the electrophysiological data points and for imposing the electromagnetic frequencies on the subject,
wherein the medical device is configured to perform a method for identifying an acupuncture point and/or a meridian of a subject comprising the following steps:
a) detecting electrophysiological data points, detected on the basis of an electrophysiological method recording electrical activity of an organ or tissue, of the subject as a function of electromagnetic frequencies imposed on the subject,
b) comparing these electrophysiological data points with a standard value for each of these electrophysiological data points,
c) identifying at least one of the frequencies from step a) as a resonance frequency at which the electrophysiological data point deviates from the standard value,
d) assigning an acupuncture point and/or a meridian to the resonance frequency, and
e) therewith individualizing for said subject a set of at least one acupuncture point and/or meridian.

13. The medical device according to claim 12, further comprising an output unit, preferably a display.

14. Use of a medical device according to claim 12, preferably a bioresonance device, for identifying an acupuncture point and/or a meridian of a subject.

15. The method according to claims 1 to 11, the medical device according to claims 12 to 13 and the use according to claim 14, wherein the subject is a human being or an animal.

## Patentansprüche

1. Verfahren zum Identifizieren eines Akupunkturpunktes und/oder eines Meridians eines Subjekts, das die folgenden Schritte umfasst:
a) Detektieren elektrophysiologischer Datenpunkte, die auf der Grundlage eines elektrophysiologischen Verfahrens zum Erfassen der elektrischen Aktivität eines Organs oder Gewebes des Subjekts als Funktion von auf das Subjekt aufgeprägten elektromagnetischen Frequenzen detektiert werden,
b) Vergleichen dieser elektrophysiologischen Datenpunkte mit einem Standardwert für jeden dieser elektrophysiologischen Datenpunkte,
c) Identifizieren mindestens einer der Frequenzen aus Schritt a) als Resonanzfrequenz, bei der der elektrophysiologische Datenpunkt von dem Standardwert abweicht,
d) Zuweisen eines Akupunkturpunktes und/oder eines Meridians zu der Resonanzfrequenz und
e) damit Individualisieren eines Satzes von mindestens einem Akupunkturpunkt und/oder Meridian für das Subjekt,
wobei das Verfahren mit einer medizinischen Vorrichtung, bevorzugt einer Bioresonanzvorrichtung, zum Identifizieren eines Akupunkturpunktes und/oder eines Meridians eines Subjekts durchgeführt wird, die die folgenden Module umfasst:
- ein Modul zum Erfassen elektrophysiologischer Datenpunkte eines Subjekts
- ein Speichermodul, das eine Datenbank von Resonanzfrequenzen und der entsprechenden Akupunkturpunkte umfasst,
- ein elektromagnetisches Frequenzgeneratormodul; und
- mindestens eine Elektrode, die sowohl zum Erfassen der elektrophysiologischen Datenpunkte als auch zum Aufprägen der elektromagnetischen Frequenzen auf das Subjekt verwendet wird.

2. Verfahren nach Anspruch 1, wobei die elektrophysiologischen Datenpunkte auf der Grundlage eines Verfahrens detektiert werden, das aus der Gruppe ausgewählt ist, umfassend Elektroenzephalographie, Elektrokardiographie, Elektrogastrographie, Elektrocochleographie, Elektronystagmographie, Elektrookulographie, Elektroretinographie, Elektromyographie und Elektroneurographie, bevorzugt Elektrokardiographie.

3. Verfahren nach Anspruch 1 oder 2, wobei die detektierten elektrophysiologischen Datenpunkte für weitere Berechnungen verwendet werden und die berechneten Datenpunkte mit einem Standardwert für diese elektrophysiologischen Datenpunkte verglichen werden.

4. Verfahren nach Anspruch 3, wobei die weitere Berechnung die Berechnung der Fläche unter einer Kurve ist und wobei die Kurve aus den detektierten elektrophysiologischen Datenpunkten zusammengesetzt ist.

5. Verfahren nach einem der vorhergehenden Ansprüche, wobei ein Elektrokardiogramm erfasst wird und die elektrophysiologischen Datenpunkte aus der Gruppe ausgewählt sind, umfassend Fläche unter der P-Q-R-S-T-U-Kurve, die Steigung des Q-R-S-Peaks und die Herzfrequenzvariabilität, bevorzugt die Fläche unter der P-Q-R-S-T-U-Kurve.

6. Verfahren nach einem der vorhergehenden Ansprüche, wobei der Standardwert durch Berechnen des Mittelwerts aller elektrophysiologischen Datenpunkte des Subjekts aus Schritt a) bestimmt wird.

7. Verfahren nach einem der vorhergehenden Ansprüche, wobei die elektrophysiologischen Datenpunkte gemäß Schritt a) als eine Funktion sequentiell angelegter Frequenzspektren detektiert werden.

8. Verfahren nach einem der vorhergehenden Ansprüche, wobei die auf das Subjekt aufgeprägten Frequenzen im Bereich von 0,1 Hz bis 100 MHz liegen.

9. Verfahren nach einem der vorhergehenden Ansprüche, wobei die bestimmten Resonanzfrequenzen ein Resonanzfrequenzmuster bilden.

10. Verfahren nach einem der vorhergehenden Ansprüche, wobei der Meridian durch Identifizieren des Akupunkturpunkts und Zuweisen des Meridians zu dem Akupunkturpunkt identifiziert wird.

11. Verfahren nach einem der vorhergehenden Ansprüche, ferner umfassend einen Schritt f) Bestimmen, ob der in Schritt d) zugewiesene Akupunkturpunkt und/oder Meridian ein zu tonisierender Akupunkturpunkt und/oder Meridian oder ein zu sedierender Akupunkturpunkt und/oder Meridian ist.

12. Medizinische Vorrichtung, bevorzugt Bioresonanzvorrichtung, zum Identifizieren eines Akupunkturpunktes und/oder eines Meridians eines Subjekts, die die folgenden Module umfasst:
- ein Modul zum Erfassen elektrophysiologischer Datenpunkte eines Subjekts,
- ein Speichermodul, das eine Datenbank von Resonanzfrequenzen und der entsprechenden Akupunkturpunkte umfasst,
- ein elektromagnetisches Frequenzgeneratormodul; und
- mindestens eine Elektrode, die sowohl zum Erfassen der elektrophysiologischen Datenpunkte als auch zum Aufprägen der elektromagnetischen Frequenzen auf das Subjekt verwendet wird,
wobei die medizinische Vorrichtung dazu konfiguriert ist, ein Verfahren zum Identifizieren eines Akupunkturpunktes und/oder eines Meridians eines Subjekts durchzuführen, das die folgenden Schritte umfasst:
a) Detektieren elektrophysiologischer Datenpunkte, die auf der Grundlage eines elektrophysiologischen Verfahrens zum Erfassen der elektrischen Aktivität eines Organs oder Gewebes des Subjekts als Funktion von auf das Subjekt aufgeprägten elektromagnetischen Frequenzen detektiert werden,
b) Vergleichen dieser elektrophysiologischen Datenpunkte mit einem Standardwert für jeden dieser elektrophysiologischen Datenpunkte,
c) Identifizieren mindestens einer der Frequenzen aus Schritt a) als Resonanzfrequenz, bei der der elektrophysiologische Datenpunkt von dem Standardwert abweicht,
d) Zuweisen eines Akupunkturpunktes und/oder eines Meridians zu der Resonanzfrequenz und
e) damit Individualisieren eines Satzes von mindestens einem Akupunkturpunkt und/oder Meridian für das Subjekt.

13. Medizinische Vorrichtung nach Anspruch 12, ferner umfassend eine Ausgabeeinheit, bevorzugt eine Anzeige.

14. Verwendung einer medizinischen Vorrichtung nach Anspruch 12, bevorzugt einer Bioresonanzvorrichtung, zum Identifizieren eines Akupunkturpunktes und/oder eines Meridians eines Subjekts.

15. Verfahren nach einem der Ansprüche 1 bis 11, medizinische Vorrichtung nach Anspruch 12 bis 13 und Verwendung nach Anspruch 14, wobei das Subjekt ein Mensch oder ein Tier ist.

## Revendications

1. Procédé permettant l'identification d'un point d'acupuncture et/ou d'un méridien d'un sujet comprenant les étapes suivantes :
a) la détection de points de données électrophysiologiques, détectés sur la base d'un procédé électrophysiologique enregistrant l'activité électrique d'un organe ou d'un tissu, du sujet en fonction de fréquences électromagnétiques imposées sur le sujet,
b) la comparaison de ces points de données électrophysiologiques avec une valeur standard pour chacun de ces points de données électrophysiologiques,
c) l'identification d'au moins l'une des fréquences de l'étape a) en tant que fréquence de résonance à laquelle le point de données électrophysiologiques s'écarte de la valeur standard,
d) l'attribution d'un point d'acupuncture et/ou d'un méridien à la fréquence de résonance, et
e) avec celles-ci, l'individualisation pour ledit sujet d'un ensemble d'au moins un point d'acupuncture et/ou méridien,
ledit procédé étant réalisé avec un dispositif médical, de préférence un dispositif de biorésonance, pour identifier un point d'acupuncture et/ou un méridien d'un sujet comprenant les modules suivants :
- un module d'enregistrement des points de données électrophysiologiques d'un sujet,
- un module de mémoire comprenant une base de données de fréquences de résonance et des points d'acupuncture correspondants,
- un module générateur de fréquences électromagnétiques ; et
- au moins une électrode servant à la fois à l'enregistrement des points de données électrophysiologiques et à imposer les fréquences électromagnétiques sur le sujet.

2. Procédé selon la revendication 1, lesdits points de données électrophysiologiques étant détectés sur la base d'un procédé choisi dans le groupe comprenant l'électroencéphalographie, l'électrocardiographie, l'électrogastrographie, l'électrocochléographie, l'électronystagmographie, l'électrooculographie, l'électrorétinographie, l'électromyographie et l'électroneurographie, de préférence l'électrocardiographie.

3. Procédé selon la revendication 1 ou 2, lesdits points de données électrophysiologiques détectés étant utilisés pour des calculs supplémentaires et lesdits points de données calculés étant comparés à une valeur standard pour ces points de données électrophysiologiques.

4. Procédé selon la revendication 3, ledit calcul supplémentaire étant le calcul de l'aire sous la courbe et ladite courbe étant assemblée à partir des points de données électrophysiologiques détectés.

5. Procédé selon l'une quelconque des revendications précédentes, un électrocardiogramme étant enregistré et lesdits points de données électrophysiologiques étant sélectionnés dans le groupe comprenant aire sous la courbe P-Q-R-S-T-U, la pente du pic Q-R-S et la variabilité de la fréquence cardiaque, de préférence l'aire sous la courbe P-Q-R-S-T-U.

6. Procédé selon l'une quelconque des revendications précédentes, ladite valeur standard étant déterminée en calculant la valeur moyenne de tous les points de données électrophysiologiques du sujet de l'étape a).

7. Procédé selon l'une quelconque des revendications précédentes, lesdits points de données électrophysiologiques selon l'étape a) étant détectés en fonction de spectres de fréquences appliqués de manière séquentielle.

8. Procédé selon l'une quelconque des revendications précédentes, lesdites fréquences imposées au sujet étant comprises dans la plage de 0,1 Hz à 100 MHz.

9. Procédé selon l'une quelconque des revendications précédentes, lesdites fréquences de résonance déterminées formant un modèle de fréquence de résonance.

10. Procédé selon l'une quelconque des revendications précédentes, ledit méridien étant identifié en identifiant le point d'acupuncture et en attribuant le méridien au point d'acupuncture.

11. Procédé selon l'une quelconque des revendications précédentes, comprenant en outre l'étape de
f) détermination pour savoir si le point d'acupuncture et/ou le méridien attribué à l'étape d) est un point d'acupuncture et/ou un méridien devant être tonifié ou un point d'acupuncture et/ou un méridien devant être mis sous sédatif.

12. Dispositif médical, de préférence un dispositif de biorésonance, permettant l'identification d'un point d'acupuncture et/ou d'un méridien d'un sujet comprenant les modules suivants :
- un module d'enregistrement des points de données électrophysiologiques d'un sujet,
- un module de mémoire comprenant une base de données de fréquences de résonance et des points d'acupuncture correspondants,
- un module générateur de fréquences électromagnétiques ; et
- au moins une électrode utilisée à la fois pour enregistrer les points de données électrophysiologiques et pour imposer les fréquences électromagnétiques sur le sujet,
ledit dispositif médical étant configuré pour mettre en oeuvre un procédé d'identification d'un point d'acupuncture et/ou d'un méridien d'un sujet comprenant les étapes suivantes :
a) la détection de points de données électrophysiologiques, détectés sur la base d'un procédé électrophysiologique enregistrant l'activité électrique d'un organe ou d'un tissu, du sujet en fonction de fréquences électromagnétiques imposées sur le sujet,
b) la comparaison de ces points de données électrophysiologiques avec une valeur standard pour chacun de ces points de données électrophysiologiques,
c) l'identification d'au moins l'une des fréquences de l'étape a) en tant que fréquence de résonance à laquelle le point de données électrophysiologiques s'écarte de la valeur standard,
d) l'attribution d'un point d'acupuncture et/ou d'un méridien à la fréquence de résonance, et
e) avec celles-ci, l'individualisation pour ledit sujet d'un ensemble d'au moins un point d'acupuncture et/ou méridien.

13. Dispositif médical selon la revendication 12, comprenant en outre une unité de sortie, de préférence un dispositif d'affichage.

14. Utilisation d'un dispositif médical selon la revendication 12, de préférence un dispositif de biorésonance, permettant l'identification d'un point d'acupuncture et/ou d'un méridien d'un sujet.

15. Procédé selon les revendications 1 à 11, dispositif médical selon les revendications 12 à 13 et utilisation selon la revendication 14, ledit sujet étant un être humain ou un animal.
